# EUROPÄISCHE PATENTANMELDUNG

(11) **EP 4 000 548 A1**
(43) Veröffentlichungstag der Anmeldung: **25.05.2022**
(21) Anmeldenummer: 21208237.4
(22) Anmeldetag: 15.11.2021
(51) Int. Cl.: A61B 18/14

(54) **VERBINDUNGSVORRICHTUNG UND MONOPOLARES KABEL FÜR MONOPOLAR UND BIPOLAR BETREIBBARE CHIRURGISCHE INSTRUMENTE, CHIRURGISCHES INSTRUMENT UND CHIRURGISCHES SYSTEM**

(30) Priorität: 20.11.2020 DE 102020130716
(71) Anmelder: Karl Storz SE & Co. KG, 78532 Tuttlingen (DE)
(72) Erfinder: Kärcher, Daniel, 78532 Tuttlingen (DE); Stefan, Jochen, 78532 Tuttlingen (DE); Unger, Tobias, 78432 Tuttlingen (DE)

(57) **Zusammenfassung**

Die vorliegende Erfindung betrifft eine Verbindungsvorrichtung und ein monopolares Kabel für monopolar und bipolar betreibbare chirurgische Instrumente, insbesondere für die Hochfrequenzchirurgie (HF-Chirurgie), sowie ein entsprechendes chirurgisches Instrument und chirurgisches System), insbesondere für die HF-Chirurgie. Ein elektrischer Anschluss der Verbindungsvorrichtung ist wahlweise für einen monopolaren Betrieb mit einem monopolaren Kabel oder für einen bipolaren Betrieb mit einem bipolaren Kabel verbindbar ausgebildet. Ein erster Kontakt und ein zweiter Kontakt des elektrischen Anschlusses sind mit einer Zubehörkupplung der Verbindungsvorrichtung elektrisch verbunden und derart isoliert ausgebildet, dass ein mit der Zubehörkupplung gekoppeltes Zubehör über die beiden Kontakte wahlweise monopolar oder bipolar betreibbar ist. Die vorliegende Erfindung betrifft ferner ein monopolares Kabel für eine derartige Verbindungsvorrichtung, ein chirurgisches Instrument mit einer derartigen Verbindungsvorrichtung sowie ein chirurgisches System mit einem derartigen chirurgischen Instrument und einem derartigen monopolaren Kabel.

## Beschreibung

### GEBIET DER ERFINDUNG

Die vorliegende Erfindung betrifft eine Verbindungsvorrichtung für monopolar und bipolar betreibbare chirurgische Instrumente, insbesondere für die Hochfrequenzchirurgie (HF-Chirurgie), und ein zugehöriges monopolares Kabel sowie ein entsprechendes monopolar und bipolar betreibbares chirurgisches Instrument und ein entsprechendes chirurgisches System.

### TECHNISCHER HINTERGRUND

Chirurgische Instrumente weisen oftmals eine Verbindungsvorrichtung, insbesondere einen Handgriff oder eine Roboterschnittstelle, und ein daran mittels einer Zubehörkupplung anbringbares zumeist als Zubehör bezeichnetes Werkzeug auf, beispielsweise eine Schere, Klemme oder dergleichen.

In der HF-Chirurgie wird bei der sogenannten Elektrotomie (auch Diathermie oder Elektrokaustik) und der Koagulation (Gerinnung) die thermische Wirkung von hochfrequentem Wechselstrom auf Gewebe ausgenutzt, um Zielgewebe zu durchtrennen und gegebenenfalls gleichzeitig oder stattdessen Blutungen zu stillen. Die chirurgischen Instrumente weisen dazu eine oder mehrere (Aktiv-) Elektroden an ihrem Zubehör (z. B. Klemme, Schere oder dergleichen) auf, über welche der hochfrequente Wechselstrom in das Zielgewebe eines Patienten eingeleitet wird. Damit am Zielgewebe der gewünschte Effekt der Elektrotomie und/oder Koagulation auftritt, wird eine hohe Stromdichte am Zielgewebe eingebracht. Daher weisen (Aktiv-) Elektroden in der Regel eine geringe Oberfläche auf und sind dazu beispielsweise nadelförmig oder klingenförmig ausgebildet, um den angelegten hochfrequenten Wechselstrom mit möglichst hoher Stromdichte in das Zielgewebe einzubringen. Es können dabei entweder monopolar oder bipolar betriebene chirurgische Instrumente zum Einsatz kommen.

Bei monopolar betriebenen chirurgischen Instrumenten ist an dem Zubehör des chirurgischen Instruments eine einzige Aktivelektrode angeordnet, über welche der hochfrequente Wechselstrom in das Gewebe eines Patienten eingeleitet wird. Damit von der einzelnen Aktivelektrode aus der Strom in das Gewebe eingeleitet werden kann, muss eine großflächige Gegenelektrode (Neutralelektrode) an dem Körper des Patienten als Gegenpol angebracht werden.

Bei bipolar betriebenen chirurgischen Instrumenten sind an dem Zubehör des chirurgischen Instruments zwei Elektroden (Aktivelektrode und Neutralelektrode) angeordnet. Dabei wird von einer ersten Elektrode (Aktivelektrode) aus der hochfrequente Wechselstrom direkt gegenüber der zweiten Elektrode (Neutralelektrode) in das Zielgewebe eingebracht.

Um den gewünschten Effekt (Elektrotomie und/oder Koagulation) am Zielgewebe zu erreichen, werden bei monopolar betriebenen chirurgischen Instrumenten höhere Spannungen zwischen der Aktivelektrode am Zubehör und der großflächigen Neutralelektrode am Körper des Patienten benötigt als bei bipolar betriebenen chirurgischen Instrumenten zwischen den beiden Elektroden am Werkzeug. Somit sind in Verbindungsvorrichtungen, insbesondere in Handgriffen, von monopolar betriebenen chirurgischen Instrumenten größere Isolations- und Kriechstrecken vorzusehen als bei bipolar betriebenen chirurgischen Instrumenten. Eine Verbindungsvorrichtung bzw. ein Handgriff eines bipolar betriebenen chirurgischen Instruments kann daher nicht für monopolar betriebene chirurgische Instrumente eingesetzt werden, da es sonst zu Fehlfunktionen und Kurzschlüssen kommen kann. Ebenso kann ein Handgriff eines monopolar betriebenen chirurgischen Instruments nicht für ein bipolar betriebenes chirurgisches Instrument eingesetzt werden.

Um Verwechslungen beim Anschließen von chirurgischen Instrumenten an einen HF-Generator zu vermeiden, sind üblicherweise Stecker und Buchsen an den Handgriffen und Kabeln jeweils formcodiert, so dass monopolare Kabel nur an Handgriffen von monopolar betriebenen chirurgischen Instrumenten und bipolare Kabel nur an Handgriffen von bipolar betriebenen chirurgischen Instrumenten angeschlossen werden können.

### ZUSAMMENFASSUNG DER ERFINDUNG

Vor diesem Hintergrund liegt der vorliegenden Erfindung die Aufgabe zugrunde, eine verbesserte und insbesondere flexibler einsetzbare Verbindungsvorrichtung sowie ein monopolares Kabel für monopolar und bipolar betreibbare chirurgische Instrumente bereitzustellen.

Erfindungsgemäß wird diese Aufgabe durch eine Verbindungsvorrichtung mit den Merkmalen des unabhängigen Patentanspruchs 1 sowie ein monopolares Kabel, ein chirurgisches Instrument und ein chirurgisches System mit den Merkmalen der weiteren unabhängigen Patentansprüche gelöst.

Demgemäß ist vorgesehen:
Eine Verbindungsvorrichtung für monopolar und bipolar betreibbare chirurgische Instrumente, insbesondere für die Hochfrequenzchirurgie umfasst ein Gehäuse, eine Zubehörkupplung und einen elektrischen Anschluss. Die Zubehörkupplung ist an dem Gehäuse angeordnet. Die Zubehörkupplung ist zum mechanischen und elektrischen Koppeln eines monopolar und zusätzlich oder alternativ bipolar betreibbaren Zubehörs ausgebildet. Der elektrische Anschluss ist an dem Gehäuse angeordnet. Der elektrische Anschluss weist einen ersten Kontakt und einen zweiten Kontakt auf. Der elektrische Anschluss ist wahlweise (für einen monopolaren Betrieb) mit einem monopolaren Kabel oder (für einen bipolaren Betrieb) mit einem bipolaren Kabel verbindbar ausgebildet. Der erste Kontakt und der zweite Kontakt sind mit der Zubehörkupplung elektrisch verbunden sowie derart isoliert ausgebildet, dass ein mit der Zubehörkupplung gekoppeltes Zubehör über die beiden Kontakte wahlweise monopolar oder bipolar betreibbar ist.

Die der vorliegenden Erfindung zugrundeliegende Idee besteht darin, durch die beiden ausreichend voneinander isoliert ausgeführten Kontakte und elektrischen Verbindungen dieser beiden Kontakte mit der Zubehörkupplung ein mit der erfindungsgemäßen Verbindungsvorrichtung ausgestattetes chirurgisches Instrument wahlweise monopolar oder bipolar betreibbar auszubilden.

Das monopolar und bipolar betreibbare chirurgische Instrument kommt bei chirurgischen Eingriffen zum Einsatz. Insbesondere in der Hochfrequenzchirurgie kann mit dem wahlweise monopolar oder bipolar betreibbaren chirurgischen Instrument Gewebe durchtrennt werden (Elektrotomie) sowie zusätzlich oder alternativ Blutungen gestillt werden (Koagulation). Das monopolar und bipolar betreibbare chirurgische Instrument kann beispielsweise ein durch einen Nutzer bzw. Bediener (z. B. Chirurg) von Hand oder ein durch einen Roboter(-arm) geführtes Instrument sein.

Die Verbindungsvorrichtung ist derart ausgebildet, dass ein damit mechanisch verbundenes Zubehör geführt werden kann. Insbesondere kann beispielsweise ein als Handgriff ausgeführtes Führungselement von einem Nutzer (z. B. Chirurg) in der Hand gehalten und somit ein mechanisch verbundenes Zubehör von Hand geführt werden. Ferner kann beispielsweise eine als Manipulatorkupplung oder Roboteraufnahme ausgeführte Verbindungsvorrichtung an einen chirurgischen Roboter angeschlossen werden und somit ein mechanisch angeschlossenes Zubehör durch den chirurgischen Roboter geführt werden.

Das Gehäuse kann integral bzw. einstückig ausgeführt sein oder aus mehreren Komponenten (z. B. Schalenelementen), die mit einander lösbar oder fest mechanisch verbunden sind, gebildet sein. Das Gehäuse kann zumindest teilweise aus einem Kunststoff, einem Metall oder einer Legierung gefertigt sein. Bevorzugt kann das Gehäuse zumindest teilweise aus einem biokompatiblen Werkstoff gefertigt sein. Das Gehäuse kann mit der Zubehörkupplung lösbar oder fest mechanisch verbunden sein. Alternativ kann das Gehäuse die Zubehörkupplung aufnehmen. Weiter alternativ kann das Gehäuse die Zubehörkupplung umfassen oder integral damit ausgebildet sein. Das Gehäuse kann mit dem elektrischen Anschluss lösbar oder fest mechanisch verbunden sein. Alternativ kann das Gehäuse den elektrischen Anschluss aufnehmen. Weiter alternativ kann das Gehäuse den elektrischen Anschluss umfassen oder integral damit ausgebildet sein.

Die Zubehörkupplung ist derart ausgeführt, dass das monopolar und zusätzlich oder alternativ bipolar betreibbare Zubehör (z. B. Schere, Klemme oder dergleichen) mit dem Gehäuse lösbar mechanisch verbindbar ist (mechanische Kopplung). Beispielsweise kann die Zubehörkupplung als Innengewinde, Außengewinde, Bajonettadapter (Bajonettverschlusses), Schnappelement (Schnappverbindung), insbesondere radial gefederter Ring, oder dergleichen ausgebildet sein, welcher/welches mit einem passenden Gegenstück an einem proximalen Ende des Zubehörs mechanisch lösbar verbunden werden kann. Zudem ist die Zubehörkupplung derart ausgeführt, dass zwischen dem lösbar mechanisch verbundenen Zubehör und der Zubehörkupplung eine elektrische Verbindung besteht (elektrische Kopplung). Die elektrische Verbindung kann über eine Stecker-Buchse-Verbindung, Kontaktplatten, Schleifkontakte und dergleichen realisiert sein. Über die elektrische Kopplung ist das Zubehör mit dem ersten Kontakt und dem zweiten Kontakt des elektrischen Anschlusses elektrisch verbunden. Dazu können geeignete Leitungen von der Zubehörkupplung innen oder außen entlang des Gehäuses zu dem elektrischen Anschluss verlaufen und diese miteinander elektrisch verbinden.

Der elektrische Anschluss ist derart ausgebildet, dass das monopolare oder das bipolare Kabel mit einem geeigneten (verbindungsvorrichtungsseitigen) elektrischen Anschlusselement daran anschließbar ist. Somit kann das monopolare/bipolare Kabel über sein geeignetes, insbesondere verbindungsvorrichtungsseitiges, elektrisches Anschlusselement einerseits lösbar mechanisch mit dem elektrischen Anschluss und andererseits elektrisch mit dem ersten Kontakt und zusätzlich oder alternativ dem zweiten Kontakt verbunden werden. Beispielsweise kann der elektrische Anschluss als Stecker, Buchse, Innengewinde, Außengewinde, Bajonettadapter, Schnappelement, insbesondere radial gefederter Ring, oder dergleichen mit integriertem ersten und zweiten Kontakt ausgebildet sein.

Der elektrische Anschluss umfasst den ersten und zweiten Kontakt. Der erste und zusätzlich oder alternativ der zweite Kontakt kann zumindest teilweise aus einem elektrisch leitfähigen Material, bevorzugt Edelstahl, Gold, Platin, Kupfer, Aluminium, Wolfram oder dergleichen sowie Kombinationen daraus gefertigt sein. Insbesondere kann der erste und zusätzlich oder alternativ der zweite Kontakt aus Edelstahl mit einer Goldbeschichtung gefertigt sein. Über den ersten Kontakt und zusätzlich oder alternativ über den zweiten Kontakt kann einerseits der eine Pol eines monopolaren Kabels mit dem elektrischen Anschluss elektrisch verbunden werden. Andererseits können über den ersten Kontakt und den zweiten Kontakt die zwei Pole eines bipolaren Kabels mit dem elektrischen Anschluss elektrisch verbunden werden. Der eine Pol bzw. die zwei Pole des monopolaren/bipolaren Kabels können über die geeigneten Leitungen mit der Zubehörkupplung elektrisch verbunden sein. Dabei sind der erste elektrische Kontakt und der zweite elektrische Kontakt derart isoliert, dass sowohl bei bipolarem Betrieb mit zwei unterschiedlichen Polen, die mit dem ersten und zweiten Kontakt elektrisch verbunden sind, als auch bei monopolarem Betrieb mit einem einzigen Pol, der mit dem ersten Kontakt und zusätzlich oder alternativ mit dem zweiten Kontakt elektrisch verbunden ist, kein elektrischer Strom zwischen den beiden Kontakten und zwischen einem der Kontakte und deren Umgebung fließen kann. Bevorzugt sind der erste und der zweite Kontakt derart isoliert ausgeführt, dass bei monopolarem Betrieb eine Spannung von 0 V [Volt] bis 5500 V und bei bipolarem Betrieb eine Spannung von 0 V bis 1000 V, weiter bevorzugt bei monopolarem Betrieb eine Spannung von 0 V bis 4300 V und bei bipolarem Betrieb eine Spannung von 0 V bis 250 V und besonders bevorzugt bei monopolarem Betrieb eine Spannung von 0 V bis 3000 V und bei bipolarem Betrieb eine Spannung von 0 V bis 190 V anliegen kann, ohne das es zu einem Kurzschluss oder Spannungsüberschlag kommt. Ferner kann die Spannungssicherheit noch weitaus höher gewährleistet sein, insbesondere auch mit Werten, deren Anwendung für den jeweiligen Anwender zugelassen ist.

Mit der erfindungsgemäßen Verbindungsvorrichtung kann schnell und aufwandsarm zwischen einem monopolar betreibbaren Zubehör und einem bipolar betreibbaren Zubehör bzw. zwischen einem monopolaren Betrieb und einem bipolaren Betrieb gewechselt werden. Es muss nicht zwischen einer Verbindungsvorrichtung für monopolaren Betrieb und einer für bipolaren Betrieb gewechselt werden. Somit kann der Nutzer (z. B. Chirurg) während eines chirurgischen Eingriffs schnell und einfach zwischen monopolarem und bipolarem Betrieb wechseln. Dadurch wird die Zeit von chirurgischen Eingriffen und somit das Risiko für Patienten verringert. Ferner wird die Anzahl an nach dem chirurgischen Eingriff zu reinigenden Teilen reduziert.

Vorteilhafte Ausgestaltungen und Weiterbildungen ergeben sich aus den weiteren Unteransprüchen sowie aus der Beschreibung unter Bezugnahme auf die Figuren der Zeichnung.

Gemäß einer Weiterbildung der vorliegenden Erfindung ist eine Kontaktisolierung zum elektrischen Isolieren des ersten Kontakts und des zweiten Kontakts voneinander vorgesehen. Die Kontaktisolierung isoliert den ersten Kontakt elektrisch von dem zweiten Kontakt. Zusätzlich isoliert die Kontaktisolierung den ersten und den zweiten Kontakt gegenüber deren Umgebung. Nur in einem jeweils vordefinierten Bereich sind der erste Kontakt und der zweite Kontakt nicht elektrisch gegenüber ihrer Umgebung isoliert, sodass dort die elektrische Verbindung mit dem monopolaren oder bipolaren Kabel bzw. mit dessen einem Pol oder beiden Polen hergestellt werden kann.

Die Kontaktisolierung kann zumindest teilweise aus einem Kunststoff oder einer Keramik gefertigt sein.

Bei einer Ausführungsform wird die Kontaktisolierung durch wenigstens einen Schlauch, insbesondere Schrumpfschlauch, gebildet. Zusätzlich oder alternativ kann die Kontaktisolierung durch wenigstens eine Beschichtung gebildet sein. Zusätzlich oder alternativ kann die Leitungsisolierung durch wenigstens ein Rohr aus Polyetheretherketon (PEEK)gebildet sein. Die Kontaktisolierung umschließt den ersten Kontakt und zusätzlich oder alternativ den zweiten Kontakt. Die Kontaktisolierung ermöglicht den wahlweisen monopolaren oder bipolaren Betrieb des chirurgischen Instruments, da ein Kurzschluss in beiden Betriebsarten effektiv verhindert wird.

Gemäß einer Weiterbildung sind eine erste Stromleitung zum elektrischen Verbinden des ersten Kontakts und eine zweite Stromleitung zum elektrischen Verbinden des zweiten Kontakts mit der Zubehörkupplung in oder an dem Gehäuse angeordnet. Es ist eine Leitungsisolierung zum elektrischen Isolieren der ersten Stromleitung und der zweiten Stromleitung voneinander und gegenüber deren Umgebung vorgesehen.

Die erste Stromleitung verbindet den ersten Kontakt des elektrischen Anschlusses mit der Zubehörkupplung. Somit kann ein Pol eines an den elektrischen Anschluss angeschlossenen monopolaren oder bipolaren Kabels mit dem monopolar oder bipolar betreibbaren Zubehör, das an bzw. mit der Zubehörkupplung gekoppelt ist, elektrisch verbunden werden.

Die zweite Stromleitung verbindet den zweiten Kontakt des elektrischen Anschlusses mit der Zubehörkupplung. Somit kann der gleiche oder ein weiterer Pol eines an den elektrischen Anschluss angeschlossenen monopolaren oder bipolaren Kabels mit dem monopolar oder bipolar betreibbaren Zubehör, das an bzw. mit der Zubehörkupplung gekoppelt ist, elektrisch verbunden werden.

Die erste und zusätzlich oder alternativ die zweite Stromleitung kann zumindest teilweise aus einem elektrisch leitfähigen Material, bevorzugt Edelstahl, Gold, Platin, Kupfer, Aluminium, Wolfram oder dergleichen sowie Kombinationen daraus gefertigt sein. Insbesondere kann die erste und zusätzlich oder alternativ die zweite Stromleitung aus Edelstahl mit einer Goldbeschichtung gefertigt sein.

Die Leitungsisolierung isoliert die erste Stromleitung elektrisch von der zweiten Stromleitung. Zusätzlich isoliert die Leitungsisolierung die erste und die zweite Stromleitung gegenüber deren Umgebung. Bei einer Ausführungsform entspricht dies einer Schaftisolierung nach außen. Insbesondere weist eine Isolierung zwischen den Polen dabei keine isolierende Wirkung nach außen auf.

Die Leitungsisolierung kann zumindest teilweise aus einem Kunststoff oder einer Keramik gefertigt sein.

Gemäß einer Ausführungsform wird die Leitungsisolierung durch wenigstens einen Schlauch, insbesondere Schrumpfschlauch gebildet. Zusätzlich oder alternativ wird die Leitungsisolierung durch wenigstens eine Beschichtung gebildet. Zusätzlich oder alternativ wird die Leitungsisolierung durch wenigstens ein Rohr gebildet, welches insbesondere Kunststoff, vorzugsweise PEEK, enthält. Die Leitungsisolierung umschließt die erste Stromleitung und zusätzlich oder alternativ die zweite Stromleitung.

Die Leitungsisolierung ermöglicht den wahlweisen monopolaren oder bipolaren Betrieb des chirurgischen Instruments, da ein Kurzschluss in beiden Betriebsarten effektiv verhindert wird. Insbesondere wird ein Kurzschluss zwischen der ersten und zweiten Stromleitung im bipolaren Betrieb und zwischen der ersten bzw. zweiten Stromleitung und deren Umgebung im monopolaren und bipolaren Betrieb effektiv verhindert. Dies ist vorteilhaft insbesondere auch im Falle einer im Betrieb am Patienten vorgesehenen Neutralelektrode gewährleistet.

Gemäß einer Weiterbildung der vorliegenden Erfindung weist die Kontaktisolierung und zusätzlich oder alternativ die Leitungsisolierung eine für Wechselströme bei monopolarem Betrieb, bevorzugt für Koagulation und zusätzlich oder alternativ Elektrotomie, ausgelegte Isolationsstrecke auf.

Die Kontaktisolierung und zusätzlich oder alternativ die Leitungsisolierung ist derart ausgebildet, dass bei monopolarem Betrieb mit Wechselstrom, insbesondere mit einer Spannung von 0 V bis 5500 V, bevorzugt von 0 V bis 4300 V und besonders bevorzugt von 0 V bis 3000 V, kein messbarer Strom zwischen dem ersten Kontakt und dem zweiten Kontakt sowie zwischen diesen und deren Umgebung bzw. zwischen der ersten Stromleitung und der zweiten Stromleitung sowie zwischen diesen und deren Umgebung fließt. Zudem ist die Kontaktisolierung und zusätzlich oder alternativ die Leitungsisolierung derart ausgebildet, dass bei monopolarem Betrieb mit Wechselstrom, insbesondere mit einer Spannung von 0 V bis 5500 V, bevorzugt von 0 V bis 4300 V und besonders bevorzugt von 0 V bis 3000 V, kein messbarer Kriechstrom an ihrer Oberfläche fließt. Ein ungewollter Kurzschluss bzw. Spannungsüberschlag kann somit besonders sicher sowohl bei bipolarem als auch bei monopolarem Betrieb des chirurgischen Instruments verhindert werden.

Gemäß einer Weiterbildung der vorliegenden Erfindung ist der elektrische Anschluss zum wahlweisen Verbinden des ersten Kontakts und des zweiten Kontakts mit einem monopolaren Kabel oder mit einem bipolaren Kabel über einen gemeinsamen Stecker ausgebildet.

Der gemeinsame Stecker ermöglicht die Kontaktierung des einen Pols des monopolaren Kabels oder der beiden Pole des bipolaren Kabels mit dem ersten Kontakt und dem zweiten Kontakt über ein einziges Element, beispielsweise einen Pin. Gleichzeitig wird durch den gemeinsamen Stecker die mechanische Verbindung zwischen dem elektrischen Anschluss der Verbindungsvorrichtung und dem geeigneten, verbindungsvorrichtungsseitigen, elektrischen Anschlusselement des monopolaren oder bipolaren Kabels durch Haftreibung hergestellt. Die Haftreibung wird durch eine Übergangspassung oder Übermaßpassung eingestellt, wobei zusätzlich eine Einstellung der Haftreibung über elastische Elemente vorgesehen sein kann. Zusätzlich oder alternativ kann die mechanische Verbindung mittels einer Kugelraste, einem Sprengring oder dergleichen hergestellt werden. Durch den gemeinsamen Stecker kann auf besonders einfache Art und Weise die mechanische und die elektrische Verbindung zwischen dem elektrischen Anschluss und dem geeigneten, verbindungsvorrichtungsseitigen, elektrischen Anschlusselement hergestellt werden.

Gemäß einer Weiterbildung ist der elektrische Anschluss als der gemeinsame Stecker ausgebildet. Der gemeinsame Stecker weist auf seiner äußeren Oberfläche entlang seiner Längsrichtung in einem ersten Kontaktbereich den ersten Kontakt, in einem Isolierungsbereich die Kontaktisolierung und einem zweiten Kontaktbereich den zweiten Kontakt auf.

Der als der gemeinsame Stecker ausgebildete elektrische Anschluss der Verbindungsvorrichtung kann in dem ersten Kontaktbereich entweder den einen Pol des monopolaren Kabels oder einen der beiden Pole des bipolaren Kabels elektrisch kontaktieren. In dem zweiten Kontaktbereich kann entsprechend entweder ebenfalls der eine Pol des monopolaren Kabels oder der andere der beiden Pole des bipolaren Kabels elektrisch kontaktiert werden.

Der als der gemeinsame Stecker ausgebildete elektrische Anschluss lässt sich nach einem chirurgischen Eingriff gemeinsam mit oder separat von der Verbindungsvorrichtung besonders einfach reinigen und sterilisieren.

Gemäß einer Weiterbildung der vorliegenden Erfindung ist der elektrische Anschluss als gemeinsame Buchse ausgebildet. Die gemeinsame Buchse weist auf ihrer inneren Oberfläche entlang ihrer Längsrichtung in einem ersten Kontaktbereich den ersten Kontakt in einem Isolierungsbereich die Kontaktisolierung und einem zweiten Kontaktbereich den zweiten Kontakt auf.

Der als die gemeinsame Buchse ausgebildete elektrische Anschluss der Verbindungsvorrichtung kann in dem ersten Kontaktbereich entweder den einen Pol des monopolaren Kabels oder einen der beiden Pole des bipolaren Kabels elektrisch kontaktieren. In dem zweiten Kontaktbereich kann entsprechend entweder ebenfalls der eine Pol des monopolaren Kabels oder der andere der beiden Pole des bipolaren Kabels elektrisch kontaktiert werden.

Der als die gemeinsame Buchse ausgebildete elektrische Anschluss ist besonders vor mechanischen Verformungen geschützt, da kein Kontaktelement (z. B. Pin) nach außen absteht und somit auch nicht versehentlich verbogen werden kann.

Gemäß einer Weiterbildung der vorliegenden Erfindung ist ferner ein Betätigungselement an dem Gehäuse angeordnet. In oder an dem Gehäuse ist ein Getriebe angeordnet. Das Getriebe ist mit dem Betätigungselement mechanisch gekoppelt. Das Getriebe ist zum Übertragen einer Bewegung des Betätigungselements auf einen Mechanismus eines über die Zubehörkupplung mechanisch verbindbaren Zubehörs, insbesondere mit einer vorbestimmten Übersetzung, ausgebildet.

Das Gehäuse kann mit dem Betätigungselement lösbar oder unlösbar mechanisch verbunden sein, wobei das Betätigungselement in mindestens einem translatorischen und zusätzlich oder alternativ rotatorischen Freiheitsgrad gegenüber dem Gehäuse verschiebbar bzw. drehbar gelagert ist. Das Getriebe ist in einem Inneren des Gehäuses oder an dessen Außenseite derart beweglich gelagert, dass eine Verschiebung bzw. Rotation in mindestens einem translatorischen und zusätzlich oder alternativ rotatorischen Freiheitsgrad gegenüber dem Gehäuse möglich ist.

Das Betätigungselement dient zur Kraft- bzw. Drehmomenteileitung und Kraft- bzw. Drehmomentweiterleitung von außen. Das Betätigungselement kann beispielsweise als Handbetätigung, über die ein Nutzer bzw. Bediener (z. B. Chirurg) von Hand eine Kraft zur Betätigung eines Zubehörs einleiten kann, oder als Betätigungskupplung für einen Roboter(-arm) ausgebildet sein.

Das Getriebe ist mit dem Betätigungselement mechanisch verbunden und nimmt von außen über das Betätigungselement eingeleitete Kraft bzw. eingeleitetes Drehmoment auf und gibt diese an den Mechanismus des angeschlossenen Zubehörs im Bereich der Zubehörkupplung weiter. Dabei kann eine von dem Betätigungselement eingeleitete Kraft in ein Drehmoment bzw. bzw. eingeleitetes Drehmoment in eine Kraft umgewandelt werden. Es kann also eine Rotation in eine Translation umgewandelt werden und umgekehrt. Mit dem Getriebe kann auch eine Übersetzung mit einem vordefinierten Übersetzungsverhältnis zwischen dem Betätigungselement und dem Mechanismus des angeschlossenen Zubehörs bzw. einem Manipulator des Zubehörs, der über den Mechanismus des Zubehörs betätigt wird, realisiert werden.

Beispielsweise kann das Betätigungselement als drehbarer Scherengriff und das Getriebe als Getriebestange, die in einer Gleithülse in dem Gehäuse translatorisch verschiebbar gelagert ist, ausgebildet sein. Der Scherengriff kann an einer entsprechend geformten Gleitfläche an einem proximalen Ende der Getriebestange anliegen, sodass eine Drehbewegung des Scherengriffs auf die Getriebestange übertragen und in eine Translation umgewandelt wird.

Über das Betätigungselement und das Getriebe können verschiedene Zubehöre mit verschiedenen Manipulatoren (z. B. Schere, Klemme usw.) mit der Verbindungsvorrichtung gekoppelt und über diese betätigt werden.

Gemäß einer Weiterbildung der vorliegenden Erfindung ist das Getriebe zumindest teilweise elektrisch leitend ausgebildet. Das Getriebe bildet zumindest teilweise entweder die erste Stromleitung und zusätzlich oder alternativ die zweite Stromleitung.

Über das Getriebe wird der erste Kontakt und zusätzlich oder alternativ der zweite Kontakt des elektrischen Anschlusses mit der Zubehörkupplung elektrisch verbunden. Beispielsweise kann das als in der Gleithülse gelagerte Getriebestange ausgebildete Getriebe zumindest teilweise die erste Stromleitung über besagte Getriebestange ausbilden. Dabei ist die Getriebestange über einen Schleifkontakt mit dem ersten Kontakt des elektrischen Anschlusses elektrisch verbunden. Zusätzlich oder alternativ kann die Gleithülse zumindest einen Teil der Leitungsisolierung, z. B. in Form einer Hülse aus Polytetrafluorethylen (PTFE), und gleichzeitig zumindest teilweise die zweite Stromleitung bilden. Dazu kann um den die Leitungsisolierung bildenden (inneren) Teil der Gleithülse eine Leitungshülse angeordnet sein, die mit dem zweiten Kontakt des elektrischen Anschlusses elektrisch verbunden ist.

Auf diese Weise kann die elektrische Verbindung zwischen dem Zubehör und dem monopolaren oder bipolaren Kabel über die Verbindungsvorrichtung mit besonders wenigen Elementen auf sehr effiziente und einfache Weise hergestellt werden.

Gemäß einer Weiterbildung der vorliegenden Erfindung ist die Zubehörkupplung zum mechanischen und elektrischen Koppeln eines als Klemme oder Schere ausgeführten Zubehörs ausgebildet.

Gemäß einer Weiterbildung der vorliegenden Erfindung weisen die Kontaktisolierung und zusätzlich oder alternativ die Leitungsisolierung jeweils eine Isolationsstrecke auf, die für Wechselströme mit einer Spannung von bis zu 5,5 kV [Kilovolt], bevorzugt bis zu 4,3 kV und besonders bevorzugt bis zu 3,0 kV ausgelegt ist.

Bei monopolarem Betrieb, insbesondere in der Elektrochirurgie, kommen Wechselströme mit einer Spannung von bis zu 5500 V oder 5,5 kV zum Einsatz. Durch die Kontaktisolierung und zusätzlich oder alternativ die Leitungsisolierung wird ein ungewollter Kurzschluss oder Spannungsüberschlag zwischen dem ersten Kontakt und dem zweiten Kontakt bzw. zwischen der ersten Stromleitung und der zweiten Stromleitung effektiv verhindert.

Weiterhin ist erfindungsgemäß vorgesehen:
Ein monopolares Kabel für monopolar und bipolar betreibbare chirurgische Instrumente, insbesondere für die Hochfrequenzchirurgie, umfasst ein generatorseitiges, elektrisches Anschlusselement, ein verbindungsvorrichtungsseitiges, elektrisches Anschlusselement und eine Überbrückung. Das generatorseitige, elektrische Anschlusselement ist zum Anschließen an einen monopolaren Ausgang eines Hochfrequenzwechselstromgenerators ausgebildet. Das verbindungsvorrichtungsseitige, elektrische Anschlusselement ist zum Anschließen an einen elektrischen Anschluss einer erfindungsgemäßen Verbindungsvorrichtung wie sie zuvor beschreiben wurde, ausgebildet. Die Überbrückung ist zum elektrischen Überbrücken des ersten elektrischen Kontakts und des zweiten elektrischen Kontakts des elektrischen Anschlusses ausgebildet.

Das generatorseitige, elektrische Anschlusselement ist ausgebildet eine mechanische, beispielsweise reibschlüssige, und elektrische Verbindung mit dem monopolaren Ausgang des Generators herzustellen.

Das verbindungsvorrichtungsseitige, elektrische Anschlusselement ist ausgebildet eine mechanische, beispielsweise reibschlüssige und elektrische Verbindung mit dem elektrischen Anschluss der Verbindungsvorrichtung herzustellen. Insbesondere kann das verbindungsvorrichtungsseitige, elektrische Anschlusselement als gemeinsame Buchse ausgebildet sein, die auf den als gemeinsamen Stecker ausgebildeten elektrischen Anschluss aufgeschoben werden kann. Alternativ kann das verbindungsvorrichtungsseitige, elektrische Anschlusselement als gemeinsamer Stecker ausgebildet sein, der in den als gemeinsame Buchse ausgebildeten elektrischen Anschluss eingesteckt werden kann.

Die Überbrückung verbindet den einen Pol des monopolaren Ausgangs des Generators mit beiden Kontakten des elektrischen Anschlusses der Verbindungsvorrichtung. Dazu überbrückt die Überbrückung die voneinander (über die Kontaktisolierung) isoliert ausgeführten Kontakte des elektrischen Anschlusses der Verbindungsvorrichtung.

Mit dem monopolaren Kabel kann ein monopolar und bipolar betreibbares chirurgisches Instrument, das die erfindungsgemäße Verbindungsvorrichtung umfasst, monopolar betrieben werden. Zum Wechsel von bipolarem Betrieb in monopolaren Betrieb braucht lediglich statt eines bipolaren Kabels das monopolare Kabel an dem elektrischen Anschluss der erfindungsgemäßen Verbindungsvorrichtung angeschlossen und mit dem monopolaren Ausgang des Generators verbunden zu werden. Ein aufwändiger Instrumentenwechsel während eines chirurgischen Eingriffs kann somit vorteilhaft vermieden werden.

Gemäß einer Weiterbildung ist die Überbrückung entweder in dem generatorseitigen, elektrischen Anschlusselement, dem verbindungsvorrichtungsseitigen, elektrischen Anschlusselement oder in einem Bereich entlang des monopolaren Kabels zwischen dem generatorseitigen, elektrischen Anschlusselement und dem verbindungsvorrichtungsseitigen, elektrischen Anschlusselement angeordnet ausgebildet.

Ist die Überbrückung in dem generatorseitigen, elektrischen Anschlusselement ausgebildet, so sind an der Überbrückung zwei elektrische Leitungen elektrisch angeschlossen. Die zwei elektrischen Leitungen verlaufen isoliert voneinander entlang des monopolaren Kabels bis zu dem verbindungsvorrichtungsseitigen, elektrischen Anschlusselement. Das verbindungsvorrichtungsseitige, elektrische Anschlusselement kontaktiert den ersten und den zweiten Kontakt des elektrischen Anschlusses der Verbindungsvorrichtung getrennt voneinander elektrisch.

Ist die Überbrückung in dem Bereich entlang des monopolaren Kabels ausgebildet, so sind entlang des monopolaren Kabels von dem generatorseitigen, elektrischen Anschlusselement zu der Überbrückung eine elektrische Leitung und von der Überbrückung zu dem verbindungsvorrichtungsseitigen, elektrischen Anschlusselement zwei elektrische Leitungen elektrisch angeschlossen. Die zwei elektrischen Leitungen verlaufen isoliert voneinander. Das verbindungsvorrichtungsseitige, elektrische Anschlusselement kontaktiert den ersten und den zweiten Kontakt des elektrischen Anschlusses der Verbindungsvorrichtung getrennt voneinander elektrisch.

Bevorzugt ist die Überbrückung in dem verbindungsvorrichtungsseitigen, elektrischen Anschlusselement ausgebildet. Dabei verläuft eine elektrische Leitung entlang des monopolaren Kabels von dem generatorseitigen, elektrischen Anschlusselement zu dem verbindungsvorrichtungsseitigen, elektrischen Anschlusselement. Das verbindungsvorrichtungsseitige, elektrische Anschlusselement kontaktiert den ersten und den zweiten Kontakt des elektrischen Anschlusses der Verbindungsvorrichtung gemeinsam elektrisch.

Durch das erfindungsgemäße monopolare Kabel mit Überbrückung, insbesondere in dem verbindungsvorrichtungsseitigen, elektrischen Anschlusselement, werden der erste Kontakt und der zweite Kontakt sicher mit dem einen Pol des monopolaren Ausgangs des Generators verbunden.

Ferner ist erfindungsgemäß vorgesehen:
Ein chirurgisches Instrument, insbesondere für die Hochfrequenzchirurgie, umfasst eine erfindungsgemäße Verbindungsvorrichtung wie sie zuvor beschreiben wurde und ein monopolar und zusätzlich oder alternativ bipolar betreibbares Zubehör. Das monopolar und zusätzlich oder alternativ bipolar betreibbare Zubehör ist über die Zubehörkupplung der Verbindungsvorrichtung elektrisch und mechanisch mit der Verbindungsvorrichtung gekoppelt.

Gemäß einer Weiterbildung der vorliegenden Erfindung ist die Verbindungsvorrichtung als Handgriff, insbesondere als beweglicher Handgriff mit einem Betätigungselement zur Betätigung eines damit gekoppelten Zubehörs, das bevorzugt als Klemme oder Schere ausgeführt ist, ausgebildet.

Alle bereits zuvor beschriebenen Weiterbildungen sind auch auf das erfindungsgemäße chirurgische Instrument übertragbar bzw. anwendbar. Alle genannten Vorteile gelten ebenso für das erfindungsgemäße chirurgische Instrument entsprechend.

Außerdem ist erfindungsgemäß vorgesehen:
Ein chirurgisches System, insbesondere für die Hochfrequenzchirurgie, umfassend ein erfindungsgemäßes chirurgisches Instrument wie zuvor beschrieben, ein Hochfrequenz-Generator (HF-Generator) und ein erfindungsgemäßes monopolares Kabel wie zuvor beschrieben. Der HF-Generator weist zumindest einen monopolaren Ausgang auf. Das monopolare Kabel ist mit dem verbindungsvorrichtungsseitigen, elektrischen Anschlusselement an dem elektrischen Anschluss der Verbindungsvorrichtung des chirurgischen Instruments und mit dem generatorseitigen, elektrischen Anschlusselement an dem monopolaren Ausgang des HF-Generators angeschlossen. Das Zubehör des chirurgischen Instruments ist über die Verbindungsvorrichtung und das angeschlossene monopolare Kabel mit dem monopolaren Ausgang des HF-Generators monopolar verbunden.

Alle bereits zuvor beschriebenen Weiterbildungen sind auch auf das erfindungsgemäße chirurgische System übertragbar bzw. anwendbar. Alle genannten Vorteile gelten ebenso für das erfindungsgemäße chirurgische System entsprechend.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

### INHALTSANGABE DER ZEICHNUNG

Die vorliegende Erfindung wird nachfolgend anhand der in den schematischen Figuren der Zeichnung angegebenen Ausführungsbeispiele näher erläutert. Es zeigen dabei:
- Fig. 1: ein Ausführungsbeispiel eines chirurgischen Instruments mit einem angeschlossenen bipolaren Kabel;
- Fig. 2: einen vergrößerten Ausschnitt aus Fig. 1;
- Fig. 3: einen vergrößerten Ausschnitt des Ausführungsbeispiels des chirurgischen Instruments aus Fig. 1 mit einem angeschlossenen monopolaren Kabel;
- Fig. 4: eine Schnittansicht eines Ausführungsbeispiels eines chirurgischen Instruments;
- Fig. 5: einen vergrößerten Ausschnitt aus Fig. 4;
- Fig. 6: eine weitere Schnittansicht des chirurgischen Instruments aus Fig. 4;
- Fig. 7: das chirurgische Instrument aus Fig. 4 mit einem angeschlossenen monopolaren Kabel gemäß einem Ausführungsbeispiel; und
- Fig. 8: ein Ausführungsbeispiel eines chirurgischen Systems.

Die beiliegenden Figuren der Zeichnung sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts Anderes ausgeführt ist - jeweils mit denselben Bezugszeichen versehen.

### BESCHREIBUNG VON AUSFÜHRUNGSBEISPIELEN

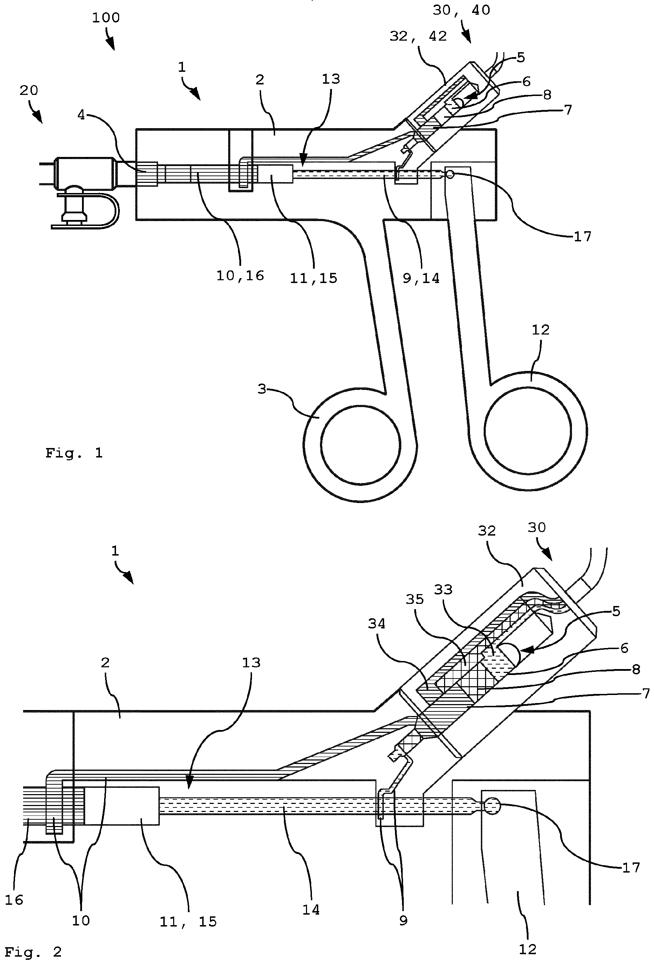

In den Figuren 1 bis 3 ist ein erstes Ausführungsbeispiel eines chirurgischen Instruments 100 mit einem angeschlossenen Kabel 30, 40 schematisch dargestellt.

Das chirurgische Instrument umfasst ein Ausführungsbeispiel einer Verbindungsvorrichtung 1 und ein Zubehör 20.

Das Zubehör 20 ist hier ein monopolar und bipolar betreibbares Zubehör. Es kann selbstverständlich auch ein nur monopolar oder nur bipolar betreibbares Zubehör an das chirurgische Instrument 100 angeschlossen werden.

Die Verbindungsvorrichtung 1 ist als Handgriff ausgebildet und umfasst ein Gehäuse 2 mit einem distalen (von dem Anwender/Chirurgen abgewandten bzw. dem Körper eines Patienten zugewandten) Griffschenkel 3, eine Zubehörkupplung 4, ein elektrisches Anschlusselement 5 mit einem ersten Kontakt 6, einem zweiten Kontakt 7, einer Kontaktisolierung 8, eine erste Stromleitung 9, eine zweite Stromleitung 10, eine Leitungsisolierung 11, ein Betätigungselement 12, das als proximaler (dem Anwender/Chirurgen zugewandter bzw. dem Körper eines Patienten abgewandter) Griffschenkel ausgebildet ist, und ein Getriebe 13.

Die Zubehörkupplung 4 ist an einem distalen Ende der Verbindungsvorrichtung angeordnet. Sie ist zum lösbaren mechanischen und elektrischen Verbinden des Zubehörs 20 mit der Verbindungsvorrichtung 1 ausgebildet. Die Zubehörkupplung 4 ist hier beispielhaft als in radialer Richtung gefederter Ring ausgebildet, der in eine Nut an der Mantelfläche eines Schafts eines Werkzeugs einrasten kann bzw. damit verrastbar ist.

Das elektrische Anschlusselement 5 ist an einem proximalen Ende der Verbindungsvorrichtung 1 angeordnet und als gemeinsamer Stecker ausgebildet. Der gemeinsame Stecker 5 weist hier beispielhaft eine im Wesentlichen zylindrische Form mit kreisrundem Querschnitt und einer sich über die Mantelfläche der Zylinderform erstreckenden äußeren Oberfläche auf. In einem ersten, hier proximalen, Bereich ist der erste Kontakt 6 auf der äußeren Oberfläche des gemeinsamen Steckers 5 angeordnet. In einem zweiten, hier distalen, Bereich ist der zweite Kontakt 7 auf der äußeren Oberfläche des gemeinsamen Steckers 5 angeordnet. Der erste Kontakt 6 und der zweite Kontakt 7 weisen jeweils eine elektrische Verbindung zum distalen Ende des gemeinsamen Steckers 5 hin auf. Der erste Kontakt 6 und der zweite Kontakt 7 sowie deren elektrische Verbindungen sind entlang des gemeinsamen Steckers 5 durch die Kontaktisolierung 8 gegeneinander elektrisch isoliert.

Die Kontaktisolierung 8 ist derart ausgebildet, dass in einem monopolaren oder bipolaren Betrieb bei einem Wechselstrom mit einer Spannung von bis zu 5,5 kV kein Kurzschluss oder Spannungsüberschlag zwischen dem ersten Kontakt 6 und dem zweiten Kontakt 7 (im bipolaren Betrieb) oder zwischen diesen und deren Umgebung (im monopolaren und bipolaren Betrieb) auftreten kann. Im monopolaren Betrieb kann der Strom lediglich von einem oder beiden der Kontakte zu einer am Patienten vorgesehenen Neutralelektrode fließen.

An dem gemeinsamen Stecker 5 ist ein Kabel 30, 40 über ein geeignetes verbindungsvorrichtungsseitiges Anschlusselement 32, 42 elektrisch angeschlossen und lösbar mechanisch verbunden. Das verbindungsvorrichtungsseitige elektrische Anschlusselement 32, 42 ist dazu als passende gemeinsame Buchse ausgebildet und auf den gemeinsamen Stecker 5 aufgeschoben.

Die erste Stromleitung 9 verbindet den ersten Kontakt 6 elektrisch mit einem über eine Getriebestange 14 beweglichen Teil der Zubehörkupplung 4. Dabei wird ein Teil der ersten Stromleitung 9 von der Getriebestange 14 des Getriebes 13 gebildet.

Die zweite Stromleitung 10 verbindet den zweiten Kontakt 7 elektrisch mit einem festen Teil der Zubehörkupplung 4. Dabei wird ein Teil der zweiten Stromleitung 10 von einer Leithülse 16 des Getriebes 13 gebildet.

Die erste Stromleitung und die zweite Stromleitung werden gegeneinander und zumindest teilweise gegenüber ihrer Umgebung durch die Leitungsisolierung 11 elektrisch isoliert. Dabei wird ein Teil der Leitungsisolierung 11 durch eine Gleithülse 15 des Getriebes 13 gebildet. Die Leitungsisolierung 11 ist derart ausgebildet, dass in einem monopolaren oder bipolaren Betrieb bei einem Wechselstrom mit einer Spannung von bis zu 5,5 kV kein Kurzschluss oder Spannungsüberschlag zwischen der ersten Stromleitung 9 und der zweiten Stromleitung 10 (im bipolaren Betrieb) oder zwischen diesen und deren Umgebung (im monopolaren und bipolaren Betrieb) auftreten kann.

Das Betätigungselement 12 ist als proximaler Griffschenkel ausgebildet. Der proximale Griffschenkel 12 ist drehbar in dem Gehäuse 2 gelagert und kann relativ zu dem distalen Griffschenkel 3 gedreht bzw. rotiert oder geschwenkt werden. Über den proximalen Griffschenkel 12 kann somit ein Drehmoment bzw. eine Drehbewegung auf das Getriebe 13 übertragen werden.

Das Getriebe 13 umfasst die Getriebestange 14 und eine Gleithülse 15 mit darauf angeordneter Leithülse 16.

Die Getriebestange 14 ist translatorisch in Richtung auf die Zubehörkupplung 4 zu verschiebbar gegenüber dem Gehäuse 2 in der Gleithülse 15 gelagert. Über eine Gleitfläche 17 an ihrem proximalen Ende ist die Getriebestange 14 mit dem proximalen Griffschenkel 12 mechanisch verbunden. Die Getriebestange 14 erstreckt sich bis zu der Zubehörkupplung 4, sodass sie dort eine Kraft bzw. eine Translation auf einen Mechanismus (hier nicht dargestellt) des angekoppelten Zubehörs 20 übertragen kann.

Die Gleithülse 15 stellt zum einen die Gleitlagerung für die Getriebestange 14 gegenüber dem Gehäuse 2 und zum anderen einen Teil der Leitungsisolierung 11 bereit. Dazu ist die Gleithülse 15 an ihrer äußeren Oberfläche von der Leithülse 16 umschlossen. Die Gleithülse 15 kann aus einem Kunststoff, insbesondere einem reibungsreduzierenden und vorzugsweise dampfsterilisierbaren Kunststoff wie PEEK, Polytetrafluorethylen (PTFE) und dergleichen gefertigt sein. Die Leithülse 17 enthält ein elektrisch leitfähiges Material, bevorzugt Edelstahl, Gold, Platin, Kupfer, Aluminium oder dergleichen, insbesondere mit Gold beschichteten Edelstahl. Dadurch ist die Getriebestange 14 linear mit geringer Gleitreibung in der Gleithülse 15 beweglich und zusätzlich gegenüber der zweiten Stromleitung 10 bzw. der elektrisch leitfähigen Leithülse 17 elektrisch isoliert.

Über die Gleitfläche 17 wird ein Drehmoment bzw. eine Drehbewegung oder Rotation des proximalen Griffschenkels 12 auf die Getriebestange 14 übertragen und in eine translatorische Kraft bzw. eine translatorische Bewegung oder Translation umgewandelt. Die Getriebestange 14 überträgt die Kraft bzw. Translation auf den Mechanismus des angekoppelten Zubehörs 20.

Somit wird neben der mechanischen Verbindung zu dem Gehäuse 2 an der Zubehörkupplung 4 einerseits eine elektrische Verbindung zwischen dem an das Gehäuse 2 gekoppelten Zubehör 20 und dem ersten Kontakt 6 sowie dem zweiten Kontakt 7 und andererseits eine mechanische Verbindung zwischen dem Mechanismus des Zubehörs 20 und dem Getriebe 13 hergestellt.

In Fig. 2 ist ein vergrößerter Ausschnitt des chirurgischen Instruments gemäß Fig. 1 mit einem angeschlossenen bipolaren Kabel 30 schematisch dargestellt.

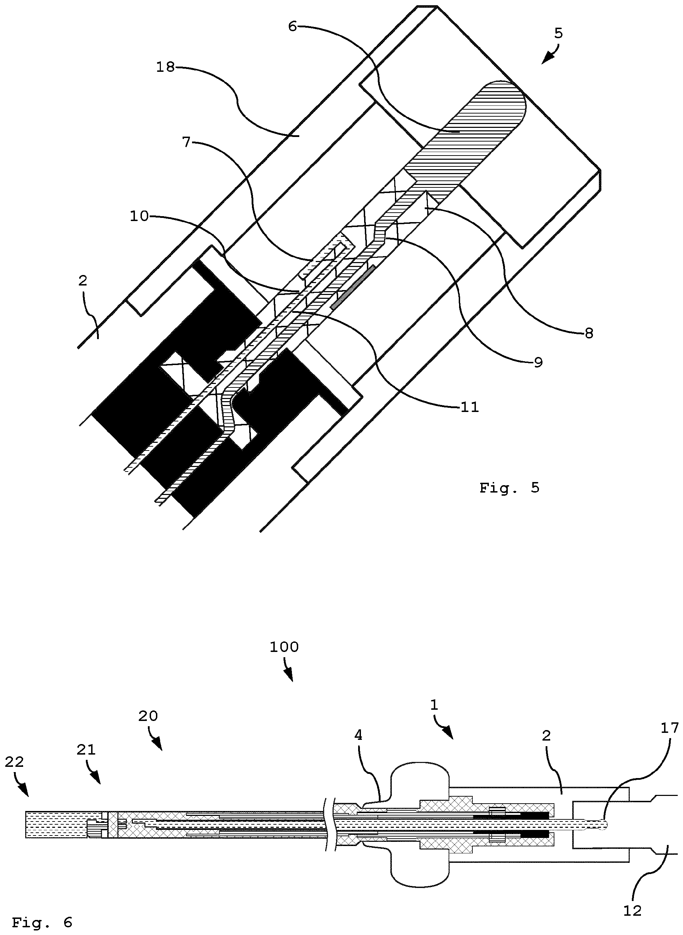

Das bipolare Kabel 30 ist bei der dargestellten Ausführungsform mit einem als gemeinsame Buchse geformten verbindungsvorrichtungsseitigen elektrischen Anschlusselement 32 auf dem gemeinsamen Stecker 5 aufgeschoben. Bei weiteren Ausführungsformen kann der gemeinsame Stecker 5 zusätzlich von einer hier zur besseren Übersichtlichkeit nicht dargestellten Steckerhülse 18 (siehe Fig. 5) mit gegenüber dem Anschlusselement 32 größerem Innendurchmesser umgeben sein, welche den Stecker 5 schützt und zur Aufnahme des als gemeinsame Buchse geformten Anschlusselements 32 des bipolaren Kabels ausgebildet ist.

Die gemeinsamen Buchse 32 weist eine Bohrung in im Wesentlichen zylindrischer Form mit kreisrundem Querschnitt auf, die zur Aufnahme des gemeinsamen Steckers 5 ausgebildet ist. Die gemeinsame Buchse 32 bildet mit dem gemeinsamen Stecker 5 eine Übergangspassung oder eine Übermaßpassung, sodass eine ausreichende Haftreibung zum lösbaren mechanischen Verbinden zwischen den beiden herrscht. Zusätzlich oder alternativ kann die mechanische Verbindung über einen O-Ring oder radial gefederten (Spreng-)Ring zwischen der gemeinsamen Buchse 32 und dem gemeinsamen Stecker 5 hergestellt werden.

In einem proximalen Bereich der gemeinsamen Buchse 32 ist entlang des inneren Umfangs der Bohrung ein erster Kabelkontakt 33 angeordnet. In einem distalen Bereich der gemeinsamen Buchse 32 ist entlang des inneren Umfangs der Bohrung ein zweiter Kabelkontakt 34 angeordnet. Zwischen dem ersten Kabelkontakt 33 und dem zweiten Kabelkontakt 34 ist eine Kabelkontaktisolierung 35 angeordnet, die den ersten Kabelkontakt 33 von dem zweiten Kabelkontakt 34 elektrisch isoliert.

Wenn die gemeinsame Buchse 32 auf den gemeinsamen Stecker 5 aufgeschoben ist, liegt eine lösbare mechanische Verbindung zwischen dem bipolaren Kabel 30 und der Verbindungsvorrichtung 1 vor. Ferner liegt der erste Kabelkontakt 33 ausschließlich an dem ersten Kontakt 6 und der zweite Kabelkontakt 34 ausschließlich an dem zweiten Kontakt 7 an, sodass zwischen diesen jeweils eine elektrische Verbindung besteht.

Über das angeschlossene bipolare Kabel 30 kann das chirurgische Instrument 100 bipolar betrieben werden.

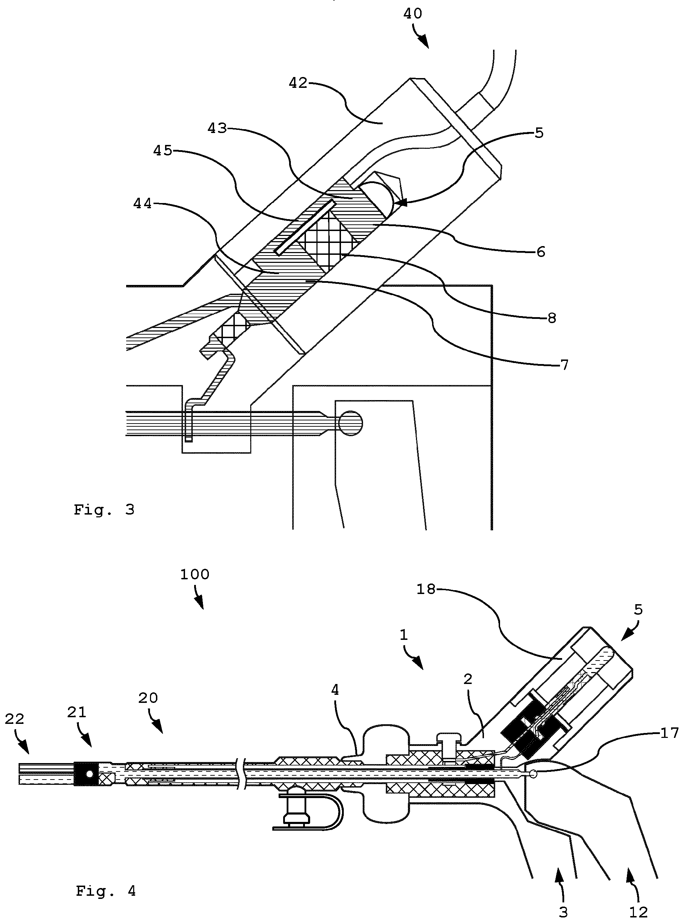

In Fig. 3 ist ein vergrößerter Ausschnitt des chirurgischen Instruments der Fig. 1 mit einem angeschlossenen monopolaren Kabel 40 gemäß einem Ausführungsbeispiel schematisch dargestellt. Es werden nachfolgend nur die Unterschiede zu dem bipolaren Kabel 30 aus Fig. 2 erläutert. Im Übrigen gilt das gleiche wie zuvor in Bezug auf Fig. 2 beschrieben.

Das monopolare Kabel 40 ist mit seinem als gemeinsame Buchse ausgeformten verbindungsvorrichtungsseitigen elektrischen Anschlusselement 42 auf den gemeinsamen Stecker 5 aufgeschoben. Die gemeinsamen Buchse 42 weist eine Bohrung in im Wesentlichen zylindrischer Form mit kreisrundem Querschnitt auf. Die gemeinsame Buchse 42 bildet mit dem gemeinsamen Stecker 5 eine Übergangspassung oder eine Übermaßpassung, sodass eine ausreichende Haftreibung zum lösbaren mechanischen Verbinden zwischen den beiden herrscht. Zusätzlich oder alternativ kann die mechanische Verbindung über einen O-Ring oder radial gefederten (Spreng-)Ring zwischen der gemeinsamen Buchse 42 und dem gemeinsamen Stecker 5 hergestellt werden.

In einem proximalen Bereich der gemeinsamen Buchse 42 ist entlang des inneren Umfangs der Bohrung ein erster Kabelkontakt 43 angeordnet. In einem distalen Bereich der gemeinsamen Buchse 42 ist entlang des inneren Umfangs der Bohrung ein zweiter Kabelkontakt 44 angeordnet. Zwischen dem ersten Kabelkontakt 43 und dem zweiten Kabelkontakt 44 ist eine Überbrückung 45 angeordnet, die den ersten Kabelkontakt 43 und den zweiten Kabelkontakt 44 elektrisch überbrückt. Alternativ kann auch nur ein einziger langgestreckter Kabelkontakt, der sich über die gleiche Fläche wie der erste und der zweite Kabelkontakt 43, 44 zusammen erstreckt, in der Bohrung angeordnet sein.

Wenn die gemeinsame Buchse 42 auf den gemeinsamen Stecker 5 aufgeschoben ist, liegt eine lösbare mechanische Verbindung zwischen dem monopolaren Kabel 40 und der Verbindungsvorrichtung 1 vor. Ferner liegt der erste Kabelkontakt 43 ausschließlich an dem ersten Kontakt 6 und der zweite Kabelkontakt 44 ausschließlich an dem zweiten Kontakt 7 an, sodass beide Kontakte 6, 7 über die Überbrückung 45 und die beiden Kabelkontakte 43, 44 des monopolaren Kabels 40 überbrückt sind.

Über das angeschlossene monopolare Kabel 40 kann das chirurgische Instrument 100 monopolar betrieben werden.

In den Figuren 4 bis 7 ist ein weiteres Ausführungsbeispiel des chirurgischen Instruments 100 gemäß der vorliegenden Erfindung schematisch dargestellt, welches im Wesentlichen dem Ausführungsbeispiel der Figuren 1 bis 3 entspricht. Es werden nachfolgend nur Unterschiede zu dem Ausführungsbeispiel der Figuren 1 bis 3 erläutert. Im Übrigen gelten die zuvor zu den Figuren 1 bis 3 gemachten Ausführungen entsprechend.

Der gemeinsame Stecker 5 weist zusätzlich eine Steckerhülse 18 auf, die den zylinderförmigen gemeinsamen Stecker 5 in einem vordefinierten radialen Abstand umgibt. Die Steckerhülse 18 schützt den gemeinsamen Stecker 5 vor mechanischer Verformung und erleichtert das Aufschieben einer gemeinsamen Buchse eines Kabels auf den gemeinsamen Stecker 5 durch ihre Führungseigenschaften. Zusätzlich kann ein O-Ring oder radial gefederter (Spreng-)Ring vorgesehen sein, der die Reibung zwischen der gemeinsamen Buchse und dem gemeinsamen Stecker 5 bzw. der Steckerhülse 18 erhöht.

Das Zubehör 20 ist hier als Klemme ausgeführt. Die Klemme 20 umfasst eine Mechanik 21 und einen Manipulator 22, der hier mit zwei Klemmbacken gebildet ist. Die Klemmbacken können über die Mechanik 21 aufeinander zu und wieder voneinander weg verdreht werden. Die Mechanik 21 ist an der Zubehörkupplung 4 mit dem Getriebe 13, insbesondere mit der Getriebestange 14 mechanisch verbunden. Die Getriebestange 14 überträgt ein Drehmoment bzw. eine Drehbewegung als translatorische Kraft bzw. Translation auf die Mechanik 21. Die Mechanik 21 wandelt die translatorische Kraft bzw. Translation in ein Drehmoment bzw. eine Drehbewegung um und überträgt diese(s) auf die Klemmbacken des Manipulators 22.

In Fig. 5 ist ein vergrößerter Ausschnitt des chirurgischen Instruments der Fig. 4 schematisch dargestellt.

Der gemeinsame Stecker 5 liegt hier geschützt innerhalb der Steckerhülse 18, die mit dem Gehäuse 2 lösbar oder fest mechanisch verbunden ist. Die Steckerhülse 18 kann auch integral bzw. einstückig mit dem Gehäuse 2 ausgeführt sein. Bei weiteren Ausführungsformen wäre auch eine Steckerhülse 18 an einem Griffschenkel, vorzugsweise dem nicht beweglichen Griffschenkel 3, denkbar.

In Fig. 6 ist das chirurgische Instrument der Fig. 4 in einer zur Fig. 4 senkrechten Schnittebene schematisch dargestellt.

An der Gleitfläche 17 wird ein Drehmoment bzw. eine Drehbewegung des proximalen Griffschenkels 12 auf die Getriebestange 14 übertragen. Durch das Zusammenspiel aus Gleitfläche 17 und in der Gleithülse 15 translatorisch geführter Getriebestange 14 wird das Drehmoment bzw. die Drehbewegung in eine translatorische Kraft bzw. Translation umgewandelt.

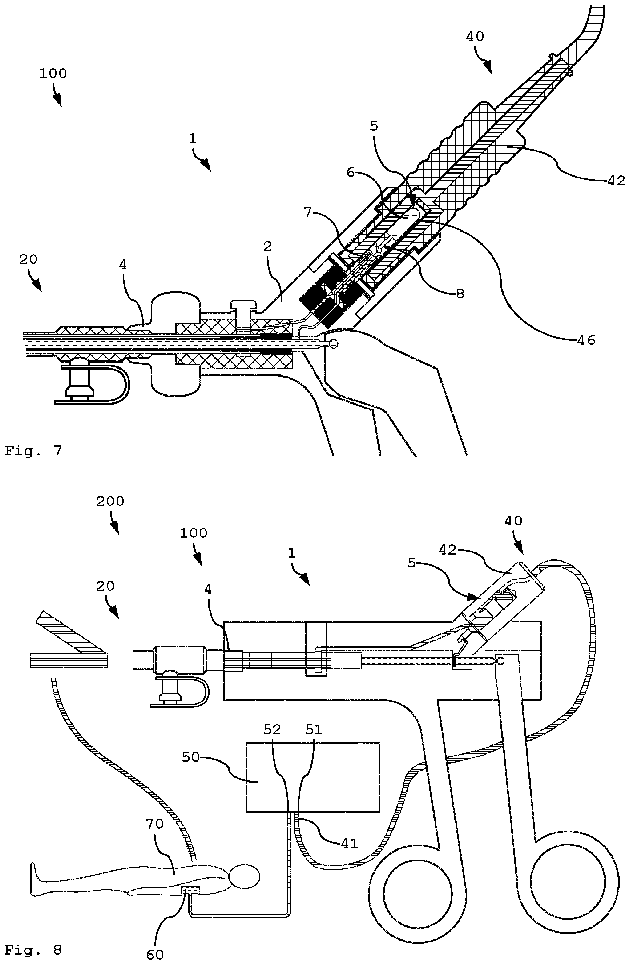

In Fig. 7 ist das chirurgische Instrument der Fig. 4 mit einem angeschlossenen monopolaren Kabel 40 gemäß einem weiteren Ausführungsbeispiel schematisch dargestellt, welches im Wesentlichen dem Ausführungsbeispiel der Fig. 3 entspricht. Es werden nachfolgend nur Unterschiede zu dem Ausführungsbeispiel der Fig. 3 erläutert. Im Übrigen gilt das gleiche wie zuvor in Bezug auf Fig. 3 beschrieben.

Das monopolare Kabel 40 weist anstelle zweier Kabelkontakte mit Überbrückung (vgl. Fig. 3) lediglich einen einzigen langgestreckten Kabelkontakt 46 in dem als gemeinsame Buchse ausgeformten verbindungsvorrichtungsseitigen elektrischen Anschlusselement 42 auf. Der Kabelkontakt 42 verbindet den ersten Kontakt 6 und den zweiten Kontakt 7 elektrisch und überbrückt sie somit.

In Fig. 8 ist ein Ausführungsbeispiel eines chirurgischen Systems 200 schematisch dargestellt.

Das chirurgische System 200 umfasst das chirurgische Instrument 100 gemäß der Figuren 1 bis 3 oder 4 bis 7, das monopolare Kabel 40 gemäß Fig. 3 oder 7, einen Hochfrequenz-Generator (HF-Generator) 50 und eine Gegenelektrode bzw. Neutralelektrode 60.

Das monopolare Kabel 40 ist mit einem generatorseitigen, elektrischen Anschlusselement 41 an einem monopolaren Ausgang 51 des HF-Generators 50 angeschlossen. Mit dem verbindungsvorrichtungsseitigen elektrischen Anschlusselement 42 ist das monopolare Kabel 40 an dem gemeinsamen Stecker 5 der Verbindungsvorrichtung 1 des chirurgischen Instruments 100 angeschlossen. Der erste Kontakt und der zweite Kontakt des gemeinsamen Steckers 5 sind durch die Überbrückung des monopolaren Kabels 40 überbrückt und mit dem einen Pol des HF-Generators 50 elektrisch verbunden. Das Zubehör 20 des chirurgischen Instruments 100 ist über die Zubehörkupplung 4 der Verbindungsvorrichtung 1 mit der Verbindungsvorrichtung 1 mechanisch und elektrisch verbunden. Dabei ist das Zubehör 20 über die Verbindungsvorrichtung 1 mit dem einen Pol des HF-Generators 50 elektrisch verbunden.

Die Gegenelektrode 60 ist über ein monopolares Kabel an einem Gegenelektrodenausgang 52 des HF-Generators 52 angeschlossen und an einem Patienten 70 angebracht, beispielsweise in Form einer Klebeelektrode. Somit ist die Gegenelektrode 60 bzw. der Patient 70 mit einem Gegenpol des HF-Generators 50 elektrisch verbunden.

Ein Chirurg kann mit dem chirurgischen Instrument 100 das über das monopolare Kabel 40 von dem Generator monopolar betrieben wird bei einem chirurgischen Eingriff Gewebe des Patienten 70 mit monopolarem Wechselstrom zwischen dem Zubehör 20 und der Gegenelektrode 60 schneiden (Elektrotomie) und gleichzeitig oder alternativ Blutungen stillen (Koagulation) .

Zum Wechsel in einen bipolaren Betrieb während des chirurgischen Eingriffs braucht lediglich statt des monopolaren Kabels 40 ein bipolares Kabel mit seinem verbindungsvorrichtungsseitigen, elektrischen Anschlusselement auf den gemeinsamen Stecker 5 aufgeschoben und mit seinem generatorseitigen, elektrischen Anschlusselement mit einem bipolaren Ausgang des HF-Generators 50 verbunden werden.

Obwohl die vorliegende Erfindung anhand bevorzugter Ausführungsbeispiele vorstehend vollständig beschrieben wurde, ist sie darauf nicht beschränkt, sondern auf vielfältige Art und Weise modifizierbar.

Beispielsweise kann statt einer Überbrückung im monopolaren Kabel auch ein Überbrücken durch eine schaltbare Überbrückung in der Verbindungsvorrichtung oder in dem HF-Generator (z. B. durch einen Schalter an der Verbindungsvorrichtung und/oder am HF-Generator) vorgesehen sein.

### BEZUGSZEICHENLISTE

- 1: Verbindungsvorrichtung
- 2: Gehäuse
- 3: proximaler Griffschenkel
- 4: Zubehörkupplung
- 5: elektrisches Anschlusselement / gemeinsamer Stecker
- 6: erster Kontakt
- 7: zweiter Kontakt
- 8: Kontaktisolierung
- 9: erste Stromleitung
- 10: zweite Stromleitung
- 11: Leitungsisolierung
- 12: Betätigungselement / distaler Griffschenkel
- 13: Getriebe
- 14: Getriebestange
- 15: Gleithülse
- 16: Leithülse
- 17: Gleitfläche
- 18: Steckerhülse
- 20: Zubehör
- 21: Mechanik
- 22: Manipulator
- 30: bipolares Kabel
- 32: verbindungsvorrichtungsseitiges elektrisches An-schlusselement
- 33: erster Kabelkontakt
- 34: zweiter Kabelkontakt
- 35: Kabelkontaktisolierung
- 40: monopolares Kabel
- 41: generatorseitiges elektrisches Anschlusselement
- 42: verbindungsvorrichtungsseitiges elektrisches An-schlusselement
- 43: erster Kabelkontakt
- 44: zweiter Kabelkontakt
- 45: Überbrückung
- 46: langgestreckter Kabelkontakt
- 50: HF-Generator
- 51: monopolarer Ausgang
- 52: Gegenelektrodenausgang
- 60: Gegenelektrode
- 70: Patient
- 100: chirurgisches Instrument
- 200: System

## Patentansprüche

1. Verbindungsvorrichtung (1) für monopolar und bipolar betreibbare chirurgische Instrumente (100), insbesondere für die Hochfrequenzchirurgie, mit:
einem Gehäuse (2);
einer Zubehörkupplung (4), die an dem Gehäuse (2) angeordnet und zum mechanischen und elektrischen Koppeln eines monopolar und/oder bipolar betreibbaren Zubehörs (20) ausgebildet ist;
einem an dem Gehäuse (2) angeordneten elektrischen Anschluss (5), der einen ersten Kontakt (6) und einen zweiten Kontakt (7) aufweist,
wobei der elektrische Anschluss (5) wahlweise mit einem monopolaren Kabel (40) oder mit einem bipolaren Kabel (30) verbindbar ausgebildet ist, und
wobei der erste Kontakt (6) und der zweite Kontakt (7) mit der Zubehörkupplung (4) elektrisch verbunden und derart isoliert ausgebildet sind, dass ein mit der Zubehörkupplung (4) gekoppeltes Zubehör (20) über die beiden Kontakte (6, 7) wahlweise monopolar oder bipolar betreibbar ist.

2. Verbindungsvorrichtung (1) nach Anspruch 1,
**dadurch gekennzeichnet,**
**dass** eine Kontaktisolierung (8) zum elektrischen Isolieren des ersten Kontakts (6) und des zweiten Kontakts (7) voneinander vorgesehen ist.

3. Verbindungsvorrichtung (1) nach Anspruch 1 oder 2, **dadurch gekennzeichnet,**
**dass** eine erste Stromleitung (9) zum elektrischen Verbinden des ersten Kontakts (6) und eine zweite Stromleitung (10) zum elektrischen Verbinden des zweiten Kontakts (7) mit der Zubehörkupplung (4) in oder an dem Gehäuse (2) angeordnet sind,
wobei eine Leitungsisolierung (11) zum elektrischen Isolieren der ersten Stromleitung (9) und der zweiten Stromleitung (10) voneinander und gegenüber deren Umgebung vorgesehen ist.

4. Verbindungsvorrichtung (1) nach einem der Ansprüche 2 oder 3,
**dadurch gekennzeichnet,**
**dass** die Kontaktisolierung (8) und/oder die Leitungsisolierung (11) eine für Wechselströme bei monopolarem Betrieb, bevorzugt für Koagulation und/oder Elektrotomie, ausgelegte Isolationsstrecke aufweist.

5. Verbindungsvorrichtung (1) nach einem der vorstehenden Ansprüche,
**dadurch gekennzeichnet,**
**dass** der elektrische Anschluss (5) zum wahlweisen Verbinden des ersten Kontakts (6) und des zweiten Kontakts (7) mit einem monopolaren Kabel (40) oder mit einem bipolaren Kabel (30) über einen gemeinsamen Stecker ausgebildet ist.

6. Verbindungsvorrichtung (1) nach Anspruch 5,
**dadurch gekennzeichnet,**
**dass** der elektrische Anschluss (5) als der gemeinsame Stecker ausgebildet ist und auf seiner äußeren Oberfläche entlang seiner Längsrichtung in einem ersten Kontaktbereich den ersten Kontakt (6) in einem Isolierungsbereich die Kontaktisolierung (8) und einem zweiten Kontaktbereich den zweiten Kontakt (7) aufweist.

7. Verbindungsvorrichtung (1) nach Anspruch 5,
**dadurch gekennzeichnet,**
**dass** der elektrische Anschluss (5) als gemeinsame Buchse ausgebildet ist, und auf ihrer inneren Oberfläche entlang ihrer Längsrichtung in einem ersten Kontaktbereich den ersten Kontakt (6) in einem Isolierungsbereich die Kontaktisolierung (8) und einem zweiten Kontaktbereich den zweiten Kontakt (7) aufweist.

8. Verbindungsvorrichtung (1) nach einem der vorstehenden Ansprüche,
**dadurch gekennzeichnet,**
**dass** ferner ein Betätigungselement (12) an dem Gehäuse (2) angeordnet ist,
wobei in oder an dem Gehäuse (2) ein Getriebe (13) angeordnet und mit dem Betätigungselement (12) mechanisch gekoppelt ist, wobei das Getriebe (13) zum Übertragen einer Bewegung des Betätigungselements (12) auf einen Mechanismus eines über die Zubehörkupplung (4) mechanisch verbindbaren Zubehörs (20) ausgebildet ist.

9. Verbindungsvorrichtung (1) nach Anspruch 8,
**dadurch gekennzeichnet,**
**dass** das Getriebe (13) zumindest teilweise elektrisch leitend ausgebildet ist und zumindest teilweise entweder die erste Stromleitung (9) und/oder die zweite Stromleitung (10) bildet.

10. Verbindungsvorrichtung (1) nach Anspruch 8 oder 9, **dadurch gekennzeichnet,**
**dass** die Zubehörkupplung (4) zum mechanischen und elektrischen Koppeln eines als Klemme oder Schere ausgeführten Zubehörs (20) ausgebildet ist.

11. Verbindungsvorrichtung (1) nach einem der vorstehenden Ansprüche,
**dadurch gekennzeichnet,**
**dass** die Kontaktisolierung (8) und/oder die Leitungsisolierung (11) jeweils eine Isolationsstrecke aufweisen, die für Wechselströme mit einer Spannung von bis zu 5,5 kV [Kilovolt], bevorzugt bis zu 4,3 kV und besonders bevorzugt bis zu 3,0 kV ausgelegt ist.

12. Monopolares Kabel (40) für monopolar und bipolar betreibbare chirurgische Instrumente (100), insbesondere für die Hochfrequenzchirurgie, mit:
einem generatorseitigen, elektrischen Anschlusselement (41), das zum Anschließen an einen monopolaren Ausgang (51) eines Hochfrequenzwechselstromgenerators (50) ausgebildet ist; und
einem verbindungsvorrichtungsseitigen, elektrischen Anschlusselement (42), das zum Anschließen an einen elektrischen Anschluss (5) einer Verbindungsvorrichtung (1) gemäß einem der vorstehenden Ansprüche ausgebildet ist, und
einer Überbrückung (45), die zum elektrischen Überbrücken des ersten elektrischen Kontakts (6) und des zweiten elektrischen Kontakts (7) des elektrischen Anschlusses (5) ausgebildet ist.

13. Monopolares Kabel (40) nach Anspruch 12,
**dadurch gekennzeichnet,**
**dass** die Überbrückung (45) entweder in dem generatorseitigen, elektrischen Anschlusselement (41), dem verbindungsvorrichtungsseitigen, elektrischen Anschlusselement (42) oder in einem Bereich entlang des monopolaren Kabels (40) zwischen dem generatorseitigen, elektrischen Anschlusselement (41) und dem verbindungsvorrichtungsseitigen, elektrischen Anschlusselement (42) ausgebildet ist.

14. Chirurgisches Instrument (100), insbesondere für die Hochfrequenzchirurgie, mit:
einer Verbindungsvorrichtung (1) nach einem der Ansprüche 1 bis 11; und
einem monopolar und/oder bipolar betreibbaren Zubehör (20), welches über die Zubehörkupplung (4) der Verbindungsvorrichtung (1) elektrisch und mechanisch mit der Verbindungsvorrichtung (1) gekoppelt ist.

15. Chirurgisches Instrument (100) nach Anspruch 14, **dadurch gekennzeichnet,**
**dass** die Verbindungsvorrichtung (1) als Handgriff, insbesondere als beweglicher Handgriff mit einem Betätigungselement (12) zur Betätigung eines damit gekoppelten Zubehörs (20), das bevorzugt als Klemme oder Schere ausgeführt ist, ausgebildet ist.

16. Chirurgisches System (200), insbesondere für die Hochfrequenzchirurgie, mit:
einem chirurgischen Instrument (100) nach Anspruch 14 oder 15;
einem HF-Generator (50), der zumindest einen monopolaren Ausgang (51) aufweist;
einem monopolaren Kabel (40) nach Anspruch 12 oder 13, das mit dem verbindungsvorrichtungsseitigen, elektrischen Anschlusselement (42) an dem elektrischen Anschluss (5) der Verbindungsvorrichtung (1) des chirurgischen Instruments (100) und mit dem generatorseitigen, elektrischen Anschlusselement (41) an dem monopolaren Ausgang (51) des HF-Generators (50) angeschlossen ist,
wobei das Zubehör (20) des chirurgischen Instruments (100) über die Verbindungsvorrichtung (1) und das angeschlossene monopolare Kabel (40) mit dem monopolaren Ausgang (51) des HF-Generators (50) monopolar verbunden ist.
